# EUROPEAN PATENT APPLICATION

(11) **EP 2 133 694 A1**
(43) Date of publication of application: **16.12.2009**
(21) Application number: 08075549.9
(22) Date of filing: 11.06.2008
(51) Int. Cl.: G01N 33/50, G01N 33/574

(54) **Method for detection of stromal epitopes**

(71) Applicant: Bayer Schering Pharma Aktiengesellschaft, 13353 Berlin (DE); Charité - Universitätsmedizin Berlin, 10117 Berlin (DE)
(72) Inventor: Menssen, Hans Dietrich, Dr., 10585 Berlin (DE); Willuda, Jörg, Dr., 13127 Berlin (DE); Duerkop, Horst, Prof. Dr., 12203 Berlin (DE)

(57) **Abstract**

The present patent application relates to a method for detection of stromal epitopes in paraffin-embedded sections and methods for *in vitro* diagnosis of diseases comprising the method for detection of stromal epitopes in paraffin-embedded sections.

## Description

The present patent application relates to a method for detection of stromal epitopes in paraffin-embedded sections and methods for *in vitro* diagnosis of diseases comprising the method for detection of stromal epitopes in paraffin-embedded sections.

Current treatment of tumour diseases, in particular hematologic malignancies, often involves large doses of rather unspecific drugs, frequently resulting in severe adverse events. Thus, modem clinical cancer research is focusing on methods to discriminate between malignant and normal tissues. Such methods should be quick, reliable and cost-effective. Also, preferably the methods are performed *in vitro* in order to avoid cost-intensive machines and in order to allow high-throughput.

An attractive approach is to detect disease-specific epitopes by immunohistochemistry in tissue specimen of patients. This detection allows diagnosis of the patient as well as the determination of a suitable treatment option. For immunohistochemistry, paraffin-embedded tissue specimen are preferred as they can be prepared quickly and cost-effectively, however, immunohistochemistry with synthetic antibodies, e.g. derived from phage display has generally been found to difficult and mainly unsuccessful.

Recently, it became clear that stromal structures, including blood vessels and extracellular matrix proteins supporting cancer growth and proliferation, are strikingly different from their normal counterparts. Neovascularization is crucial for tumor growth beyond 2 mm³, and newly formed tumor blood vessels were found to be structurally and functionally altered in comparison to blood vessels of normal mature organs (Folkman et al, 1992). Vascular shunts, uneven vessel diameters, unusual fenestration with wide inter-endothelial junctions, discontinuous basement membranes (Dewhirst et al, 1989; Cohen, 2007), and dysfunctional or lack of pericytes (Eberhard et al, 2000; Morikawa et al, 2002), are key features of tumor blood vessels rendering them leaky. These characteristics may not be specific for tumor blood vessels, since there is evidence of similar changes in blood vessels associated with periodic tissue remodeling (Cohen, 2007; De Candia et al, 1999), chronic inflammatory disease (Kriegsmann et al, 2004), and severe atherosclerosis (Matter et al, 2004). However, targeting anticancer compounds selectively to epitopes that are typically expressed or over-expressed in tumor blood vessels or stroma is attractive for two reasons: (i) tumor blood vessel or stromal epitopes are stably expressed, and (ii) such epitopes are typically found in various kinds of tumors. The extra domain B of fibronectin (ED-B FN) represents such a stromal tumor tissue target (Zardi et al, 1987).

It is object of the present invention to provide a method for immunohistochemistry on paraffin-embedded tissue specimen, which allows for detection of stromal epitopes and which is also suitable for synthetic antibodies, antibody fragments and antibody mimetics.

The present invention therefore relates to a method for detection of at least one stromal epitope and/or stromal protein, wherein
a) at least one tissue specimen is prepared as paraffin sections, and
b) at least one epitope is detected by immunohistochemistry
**characterized in that**
step b) is performed by
i) steam pressure cooking over more than about 3 minutes, and
ii) in the presence of EDTA.

Preferably, the result obtained in this method is compared to at least one control tissue specimen.

The steps of steam pressure cooking over more than about 3 minutes, and in the presence of EDTA represent the phase of antigen-retrieval in the immunohistochemistry procedure, which is followed by binding and detection of an antibody, antibody fragment or antibody mimetic.

Surprisingly, the inventors found that using the method of the invention, equivalent immunohistological staining results on paraffin sections as compared with the immunohistological results on cryostat sections, were obtained, while preserving tissue histology. The loss of ED-B FN staining in tissue sections after addition of soluble recombinant ED-B FN to the anti-ED-B FN reagents surprisingly demonstrated the specificity of the immunohistochemical procedure. Using the method of the invention, ED-B FN expression on normal lymphoid and hematopoietic tissue and in a large series of lymphoid and hematologic malignancies, including decalcified bone marrow biopsies could be shown. Expression of ED-B FN could be found in almost all examined lymphoma samples, irrespective of their histopathologic classification.

Paraffin sections can be obtained by methods known to the skilled person. Typically, animal and human tissue samples are obtained following standard surgical procedures, such as open surgery, minimal invasive surgery, surgery following local anesthesia, needle and fine-needle biopsies. Tissue samples obtained by these means are immediately placed into routine fixation solution, preferably containing formaldehyde as the major fixative dissolved in phosphate-buffered isotone saline solution. For bone or bone marrow biopsies, a routine decalcification procedure with EDTA buffers is preferably performed during tissue fixation. Fixed tissue samples are dehydrated, preferably with ethanol and xylol by methods known to the skilled person. Subsequently, they can placed preferably into small molds and are covered with paraffin. The paraffin embedded tissue samples are typically allowed to cool down at room temperature before performing IHC. Routine paraffin sections (5-10 µm) can be cut from these tissues by a microtom.

After paraffin-embedding, the IHC is performed wherein in a first step antigens have to be retrieved, using an antigen-retrieval solution.

In a preferred embodiment, EDTA is present in the antigen retrieval solution at a concentration of about 0.5 mM to 5 mM EDTA, more preferred of about 1 to 4 mM EDTA, even more preferred of about 1.5 to 2.5 mM EDTA, and most preferred of about 2 mM EDTA.

Preferably, steam pressure cooking is performed at maximum heat for about 1 to about 10 minutes, more preferably about 2 to 9 minutes, particularly preferred about 3 to 8 minutes, even more particularly preferred about 4 to 6 minutes. In particular it is performed for about 5 minutes.

Preferably, the temperature during steam pressure cooking is about 110 to 135°C, more preferably about 120°C to 130°C, most preferably about 125 °C. Preferably is about 50 to 150 kPA, more preferably about 100 kPa (15 psi).

Preferably the pH is about 5 to 10, more preferred about 7 to 8.

The antibody may be detected by methods known to a skilled person. Either the antibody is itself labelled, eg. by a fluorescence label or secondary or tertiary detection systems may be used, like labelled secondary antibodies directed the first antibodies, wherein the secondary antibody may be labelled, or carries an enzyme useful for detection.

Tissues which are suitable for immunohistochemistry according to the present invention include all human and mammalian normal or disease-affected solid tissues.

The stromal epitope is an epitope present in a stromal protein specifically present at or enriched in, or over-expressed in the stroma of an animal. The animal may be human or non-human, preferably human.

In a preferred embodiment the molecule specifically present at or enriched in or over-expressed in the stroma is a protein, in particular an extracellular matrix protein.

Preferably the stromal protein is a "marker" protein for a disease or disorder. Preferably, the presence or increased presence or absence or reduced presence of the epitope is indicative for the disease.

In a preferred embodiment, the invention therefore relates to a method of *in vitro* diagnosis of a disease, comprising performing the method of immunohistochemistry as described above, wherein the tissue specimen is obtained from a patient, and wherein the presence or increased presence or absence or reduced presence of the stromal protein comprising the epitope is indicative for the disease.

Particularly preferred are extracellular matrix proteins which are specifically present at or enriched at or over-expressed at the site of disease.

In a particular embodiment, stromal proteins comprising the stromal epitope, especially the extracellular matrix proteins, are stratifying markers. Such stratifying markers are preferably indicative for patients either responsive to or non-responsive to a therapeutic treatment, in particular therapeutic treatment using one or more active compounds.

Therefore, in a further embodiment, the present invention relates to a method of determining whether a patient is likely to respond to a treatment, wherein a method of *in vitro* diagnosis as described above is performed, and wherein the stromal protein comprising the epitope is a stratification marker for the treatment.

In an especially preferred embodiment such active compound(s) for therapeutic treatment bind(s) to and/or act(s) on the stromal protein comprising the epitope.

Therefore, in a preferred embodiment, the present invention relates to a method of determining whether a patient is likely to respond to a treatment, wherein a method of *in vitro* diagnosis as described above is performed, and wherein the active compound used in the treatment binds to and/or acts on the stromal protein comprising the epitope.

In another embodiment, the present invention relates to a method of treatment of a patient comprising the steps
i) performing the method of *in vitro* diagnosis as described above wherein the stromal protein is a stratification marker for responsiveness to an active compound,
ii) identification of a patient who is likely to respond to the active compound, and
iii) administration of a therapeutically active amount of the active compound.

In another preferred embodiment, the present invention relates to a method of treatment of a patient comprising the steps
iv) performing the method of *in vitro* diagnosis as described above wherein the stromal protein is a stratification marker for responsiveness to an active compound, and
v) identification of a patient who is likely to respond to the active compound which binds to and/or acts on the stromal protein comprising the epitope,
   and
vi) administration of a therapeutically active amount to the active compound which binds to and/or acts on the stromal protein comprising the epitope.

In a particularly preferred embodiment, the stroma proteins are specifically present at or enriched at or over-expressed in the stroma of tumour patients, in particular in stromal proteins surrounding tumour blood vessels.

Particularly suitable proteins of the extracellular matrix are Tenascin isoform C (Tenascin C), ED-A fibronectin (ED-A FN) and ED-B fibronectin (EDB FN). Therefore, the stromal epitope is preferably part of an extracellular protein, in particular selected from ED-B fibronectin, ED-A fibronectin and Tenascin C. (Kaspar et al, 2006, Silacci et al, 2006).

FNs are high molecular-weight extracellular matrix (ECM) components abundantly expressed in a range of healthy tissues and body fluids. Various different FN isoforms can be generated due to alternative splicing at the level of the primary transcript. The ED-B, a small domain of 91 amino acids, which is identical in sequence in mouse and man, is usually absent in both plasma and tissue-fibronectin, except for some blood vessels of the regenerating endometrium and the ovaries (Alessi et al, 2004). Increased binding affinity and valence of recombinant antibody fragments lead to improved targeting of tumor-associated angiogenesis (Viti et al, 1999). However, it may become inserted in the fibronectin molecule during active tissue remodeling associated with neo-angiogenesis, thereby accumulating around the neo-vasculature and in the stroma of malignant tumors and in other tissues undergoing remodeling and angiogenesis. Several antibodies specific for the ED-B domain of fibronectin are known in the prior art. In particular, the human single chain Fv antibody fragment scFv(L19), which displays a picomolar binding affinity for ED-B, has been verified to selectively target tumor neovasculature, both in experimental tumor models (Viti et al, 1999) and in patients with cancer (Santimaria et al, 2003). It was recently shown that considerable but variable vessel-associated ED-B expression was detected in various hematologic malignancies including Hodgkin and Non-Hodgkin lymphoma (Sauer et al, 2006).

Using the method of the invention on paraffin-embedded tissues, ED-B FN expression was found in biopsies from more than 200 Hodgkin and Non-Hodgkin lymphoma (NHL) patients of nearly all entities, and in 24 patients with acute and chronic myeloproliferative diseases. ED-B FN expression was nearly absent in normal lymph nodes (n=10) and bone marrow biopsies (n=9). The extent of vascular ED-B FN expression in lymphoma tissues was positively correlated with grade of malignancy. ED-B FN expression was enhanced in lymph nodes with severe lymphadenopathy and in some hyperplastic tonsils. The method of invention is therefore highly reliable and reproducible and fulfills the requirements for *in vitro* diagnostic methods to be applied for humans and animals.

In an especially preferred embodiment, the immunohistochemistry according to the present invention is therefore performed using an antibody or antibody fragment or antibody mimetic specifically binding to the epitope and/or stromal protein. An antibody or antibody fragment or antibody mimetic may also comprise effectors like a diagnostic label or therapeutically active moiety, like a cytokine. Examples are ¹³¹I-L19-SIP and L 19-IL2.

In an especially preferred embodiment, the antibody or antibody fragment or antibody mimetic is specifically binding to ED-B fibronectin, in particular to the ED-B domain of ED-B fibronectin.

Preferred antibodies and antibody fragments specifically binding to ED-B fibronectin are disclosed in WO 2007/054120. Particularly preferred are MOR3255 and MOR3257 described in WO 2007/054120. In particular, MX1 can be used, which is MOR 3255 disclosed in WO 2007/054120 in IgG format. In particular, MX1 has the CDR sequences of MOR3255, but is in IgG format. These antibodies may be used in any antibody format or be a tagged antigen binding domain.

Further preferred monoclonal antibodies specifically recognising the ED-B-fibronectin domain as epitope are described in WO 97/45544.

A particularly preferred antibody is monoclonal antibody L19, described in WO 99/58570.

WO 01/62298 on page 8, line 12 refers to the L19 VH and L19 VL domain sequences described in Pini et al. (1998). Pini et al. describe parts of the sequence of L 19 in Table II on page 21772. L19 has the EMBL accession Number AJ 006113.

Another antibody which may be used is BC-1 (Kaczmarek et al, 1994).

L19 may be used in any format. In particular, L19-SIP described in WO 03/076469 may be used. "SIP" stands for small immunoprotein. L19-SIP is an antibody format wherein the L19-scFv is linked to an εs2-CH4 domain of IgE, and wherein two monomeric chains form a homodimer covalently linked by an S-S bridge (see e.g. WO03/076469; Borsi et al., 2002). CH4 is the domain that allows dimerization in the IgE molecule and the εs2- isoform contains a cysteine at the carboxyterminal end, which stabilizes the IgE-dimer through an inter-chain disulphide bond. In the final SIP molecule of L19-SIP the scFv(L 19) is connected to the εs2CH4 domain by a GGSG linker. The disclosure of WO03/076469 is included by reference.

In particular, the L19-antibody is in scFv format, as described in WO99/58570 or in scFv format with tags as described in WO 03/055917. In particular AP38 or AP39 described in WO 03/055917 can be used according to the invention. Also fusion proteins of L19 can be used according to the invention, like L19-IL2 and L19-TNFalpha. Such fusion proteins are disclosed in WO 01/62298. The disclosure of the fusion proteins is hereby incorporated by reference.

In another preferred embodiment, the antibody or antibody fragment or antibody mimetic is specifically binding to ED-A fibronectin. Preferred antibodies or antibody fragments are F8, B7 and D5 in disclosed in Villa A et al. (2008) and Rybak et al. (2007).

In another preferred embodiment the antibody or antibody fragment or antibody mimetic is specifically binding to Tenascin C. A preferred antibody or antibody fragment is G11 disclosed in Silacci M et al. (2006).

In a preferred embodiment, the immunohistochemistry (IHC) is performed with an antibody, in particular a synthetic antibody, more preferably an antibody fragment or antibody mimetic binding to a stromal epitope and/or stromal protein, and patients for which IHC on paraffin-embedded tissue results in strong or enhanced signal, are treated with at least one active compound, wherein the active compound also comprises a molecule, preferably an antibody or antibody fragment or antibody mimetic binding to the stromal epitope and/or stromal protein. In an especially preferred embodiment the epitope is selected from ED-A FN, ED-B FN and Tenascin C. For example, IHC can be performed with L19(scFv) or L19-IL2 or L19-SIP or ^{99Tm}Tc-AP39 or L19-TNFalpha, and patients for which IHC on paraffin-embedded tissue results in strong or enhanced signal are then treated for example with ¹³¹I-L19-SIP, or L19-IL2 or L19-TNFalpha.

In a preferred embodiment, the tumour is a hematologic malignancy, in particular a lymphoma, like Hogdkin Lymphoma or Non-Hodgkin Lymphoma (NHL), or a myeloproliferative disease.

"Specifically binding" or "specifically recognizing" as used herein refers to binding to the corresponding target. Typically, the binding molecule, antibody, antibody fragment or antibody mimetic binds with an affinity of at least about 1x10⁻⁷ M, preferably of at least about 1x10⁻⁹ M, and binds to the predetermined target with an affinity that is at least two-fold greater than its affinity for binding to a non-specific target (e.g. BSA, casein) other than the predetermined target or a closely-related target.

Surprisingly, the method of invention was found to be useful for synthetic antibodies, in particular antibody fragments and antibody mimetics, most preferred an antibody fragments. "Synthetic antibody" according to the present invention is understood as an antibody which is not naturally generated in mammalians and other animals by immunization, but by standard in vitro and in vivo methodologies of modem antibody engineering technologies. Synthetic antibody molecules are binding molecules which are based on genetically modified immunoglobulin molecules or alternative folds and are preferably isolated from large diverse collections of such binding molecules by phage display, ribosomal display etc. They may have undergone further modifications for increased binding, efficacy and optimization of the molecular format such as reformatting, changes to glycosylation, modification or elimination of the Fc part.

"Antibody" as used herein encompasses full length antibodies, comprising native antibodies, monoclonal antibodies, polyclonal antibodies and multispecific antibodies (e.g., bispecific antibodies), human antibodies, humanized antibodies, chimeric antibodies, and full IgG antibodies, as well as antibody fragments.

The term "antibody fragment" refers to a portion of a full length antibody, in which a variable region or a functional capability is retained, namely the specific binding to the target. Examples of antibody fragments include, but are not limited to, a Fab, Fab', F(ab')2, Fd, Fv, scFv, and scFv-Fc fragment, a diabody, a linear antibody, small immunoprotein formats, a single-chain antibody, a minibody, a diabody formed from antibody fragments, and multispecific antibodies formed from antibody fragments. Antibody fragments are usually smaller than full antibodies. Thereby, the pharmacokinetics are different and some antibody fragments only consist of one polypeptide chain, which can make production easier. Preferably, the antibody fragment is in scFv, (scFv)₂, or small immunoprotein format. The small immunoprotein format can be a format based on a CH3-domain (for example described in US 5,837,821) or εₛ₂CH4-domain of human IgE (for example described in WO 03/076469).

The term "monoclonal antibody" (mAb) refers to an antibody obtained from a population of substantially homogeneous antibodies; that is, the individual antibodies comprising the population that are identical except for naturally occurring mutations that may be present in minor amounts. Monoclonal antibodies are highly specific, being directed against a single antigenic determinant, also referred to as an epitope. The modifier "monoclonal" is indicative of a substantially homogeneous population of antibodies directed to the identical epitope and is not to be construed as requiring production of the antibody by any particular method. Monoclonal antibodies can be made by any technique or methodology known in the art; for example, the hybridoma method first described by Koehler et al. (1975), or recombinant DNA methods known in the art (see, e.g., U.S. Patent No. 4,816,567). In another example, monoclonal antibodies can also be isolated from phage antibody libraries, using techniques described in Clackson et al. (1991), and Marks et al. (1991).

In contrast, the antibodies in a preparation of polyclonal antibodies are typically a heterogeneous population of immunoglobulin isotypes and/or classes and also exhibit a variety of epitope specificity.

The term "chimeric" antibody as used herein is a type of monoclonal antibody in which a portion of or the complete amino acid sequence in one or more regions or domains of the heavy and/or light chain is identical with, homologous to, or a variant of the corresponding sequence in a monoclonal antibody from another species or belonging to another immunoglobulin class or isotype, or from a consensus sequence.

Certain types of antibody fragments can be generated by enzymatic treatment of a full-length antibody. Papain digestion of antibodies produces two identical antigen-binding fragments called "Fab" fragments, each with a single antigen-binding site, and a residual "Fc" fragment, so called because of its ability to crystallize readily. The Fab fragment also contains the constant domain of the light chain and the CH1 domain of the heavy chain. Pepsin treatment yields a F(ab')₂ fragment that has two antigen-binding sites and is still capable of cross-linking antigen.

Fab' fragments differ from Fab fragments by the presence of a few additional residues at the C-terminus of the CH1 domain, including one or more cysteines from the antibody hinge region. Fab-SH is the designation herein for a Fab' in which the cysteine residue(s) of the constant domains bear a free thiol group. F(ab')₂ antibody fragments are pairs of Fab' fragments linked by cysteine residues in the hinge region. Other chemical couplings of antibody fragments are also known.

"Fv" is a minimum antibody fragment that contains a complete antigen-recognition and binding site consisting of a dimer of one heavy and one light chain variable domain in tight, non-covalent association. In this configuration, the three CDRs of each variable domain interact to define an antigen-binding site on the surface of the VH VL dimer. Collectively, the six CDRs confer antigen-binding specificity to the antibody.

A "single-chain Fv" or "scFv" antibody fragment is a single chain Fv variant comprising the VH and VL domains of an antibody, in which the domains are present in a single polypeptide chain and which is capable of recognizing and binding antigen. The scFv polypeptide optionally contains a polypeptide linker positioned between the VH and VL domains that enables the scFv to form a desired three-dimensional structure for antigen binding (see, e.g., Pluckthun, 1994).

The term "diabodies" refers to small antibody fragments having two antigen-binding sites. Each fragment contains a heavy chain variable domain (VH) concatenated to a light chain variable domain (VL). By using a linker that is too short to allow pairing between the two domains on the same chain, the linked VH-VL domains are forced to pair with complementary domains of another chain, creating two antigen-binding sites.

Diabodies are described more fully, for example, in EP 404,097; WO 93/11161; and Hollinger et al. (1993).

A humanized antibody or a humanized antibody fragment includes an immunoglobulin amino acid sequence variant, or fragment thereof, which is capable of binding to a predetermined antigen, and which comprises one or more framework regions (FRs) having substantially the amino acid sequence of a human immunoglobulin and one or more CDRs having substantially the amino acid sequence of a non-human immunoglobulin. This non-human amino acid sequence is referred to herein as an "import" sequence, which is typically taken from an "import" antibody domain, particularly a variable domain. In general, a humanized antibody includes at least the complementarity determining regions (CDRs) or hypervariable loops (HVLs) of a non-human antibody, inserted between the FRs of a human heavy or light chain variable domain.

"Native antibodies" are defined herein as heterotetrameric glycoproteins, typically of about 150,000 daltons, composed of two identical light (L) chains and two identical heavy (H) chains. Each light chain is covalently linked to a heavy chain by one disulfide bond to form a heterodimer. The heterotetramer is formed by covalent disulfide linkage between the two identical heavy chains of such heterodimers. Although the light and heavy chains are linked together by one disulfide bond, the number of disulfide linkages between the two heavy chains varies by immunoglobulin isotype. Each heavy and light chain also has regularly spaced intrachain disulfide bridges. Each heavy chain has at the amino-terminus a variable domain (VH), followed by three or four constant domains (CH1, CH2, CH3, and CH4), as well as a hinge region between CH1 and CH2. Each light chain has two domains, an amino-terminal variable domain (VL) and a carboxyterminal constant domain (CL). The VL domain associates non-covalently with the VH domain, whereas the CL domain is commonly covalently linked to the CH1 domain via a disulfide bond. Particular amino acid residues are believed to form an interface between the light and heavy chain variable domains (Chothia et al., 1985). The term "hypervariable" refers to the fact that certain sequences within the variable domains differ extensively in sequence among antibodies and contain residues that are directly involved in the binding and specificity of each particular antibody for its specific antigenic determinant. Hypervariability, both in the light chain and the heavy chain variable domains, is concentrated in three segments known as CDRs or HVLs. CDRs are defined by sequence comparison in Kabat et al. (1991), whereas HVLs are structurally defined according to the three-dimensional structure of the variable domain, as described by Chothia and Lesk (1987).

Where these two methods result in slightly different identifications of a CDR, the structural definition is preferred. As defined by Kabat, CDR-L1 is positioned at about residues 24-34, CDR-L2, at about residues 50-56, and CDR-L3, at about residues 89-97 in the light chain variable domain; CDR-H1 is positioned at about residues 31-35, CDR-H2 at about residues 50-65, and CDR-H3 at about residues 95-102 in the heavy chain variable domain.

The term "label" refers to a detectable compound or composition that is conjugated directly or indirectly to the antibody or antibody fragment or antibody mimetic binding to the stroma epitope.

"Antibody mimetics" are understood as binding molecules based on protein frameworks ("scaffolds") which specifically bind to the target and which are distinct from antibodies and antibody fragments. Such scaffolds are described in Binz et al. (2005). Antibody mimetics specifically binding to ED-B fibronectin are described in Grabulovski et al. (2007).

Figure Legend:

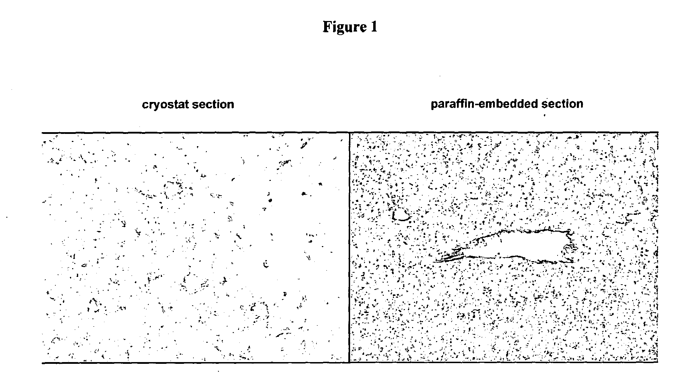

Fig. 1: ED-B FN-binding MX1 and the fusion protein L19IL2 were used for IHC on paraffin-embedded tissue. Both anti-ED-B FN constructs detected the same ED-B FN expression pattern in paraffin-embedded and frozen tissue specimens of renal carcinoma (n=6), lymphocytic lymphoma (CLL; n=2), mantle cell lymphoma (n=6), and follicular lymphoma (n=3). Identical ED-B FN expression patterns were also found in the paraffin-embedded half and the frozen half of the split F9 tumor tissue specimens.

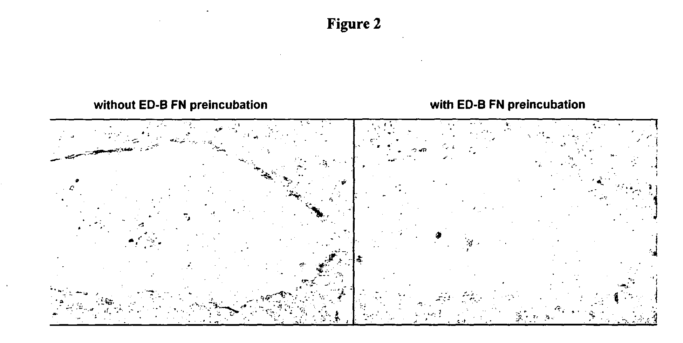

Fig. 2: MX1 and the fusion protein L19IL2 were used for IHC on paraffin-embedded tissue, however, these reagents were pre-incubated with an excess of recombinant ED-B FN. The mixtures were incubated on human clear cell renal cell carcinomas and murine F9 teratocarcinoma with subsequent immunohistological detection of their binding patterns. Recombinant ED-B FN was capable of inhibiting the immunohistological binding of these reagents.

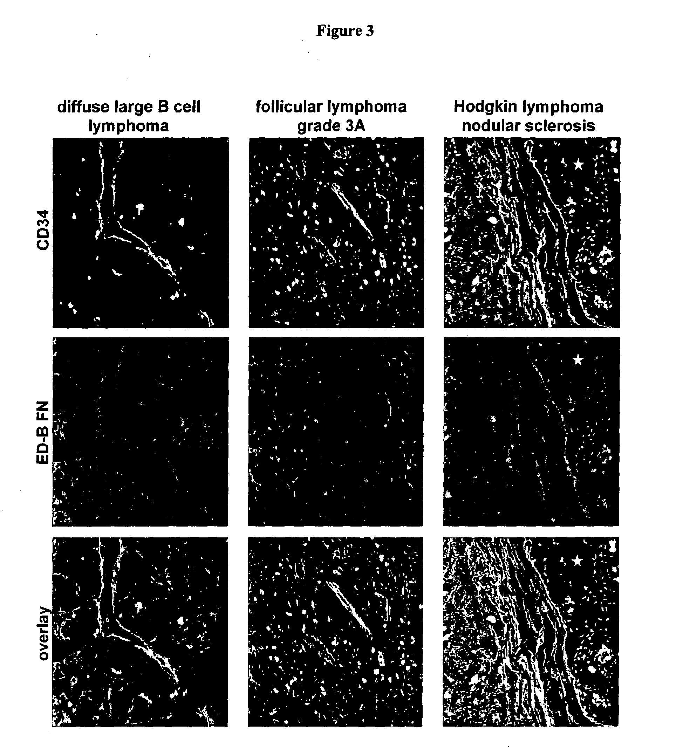

Fig. 3: Double stainings of diffuse large B cell lymphoma, marginal-zone lymphoma, lymphocytic lymphoma, plasmacytoma, follicular lymphoma (grade 1 and 3), and classical Hodgkin lymphoma (mixed cellularity and nodular sclerosis) with ED-B FN binding MX1 and a CD34 antibody revealed that ED-B FN is mostly co-localized with blood vessels, which were identified by their CD34-positive endothelia.

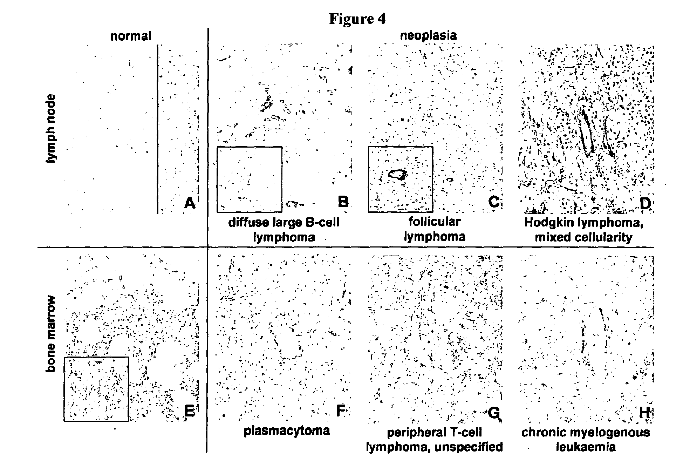

Fig. 4: Blood vessels of normal lymphoid tissue rarely and weakly express ED-B FN. ED-B FN expression of paraffin-embedded specimens of normal lymph node (A; magnification: 100x, left insert: 20x), and lymph node biopsies of diffuse large B-cell lymphoma (B; 250x, insert 20x); follicular lymphoma grade 2 (C; 20x, insert 100x), and classical Hodgkin lymphoma of mixed cellularity subtype (D; 150x). ED-B FN expression of bone marrow biopsies of normal bone marrow (E; 50x, insert 200x), multiple myeloma (F; 100x), unspecified peripheral T-cell lymphoma (G; 200x), and chronic myelogenous leukaemia (H; 200x). All ED-B FN stainings were performed with the MX1 antibody.

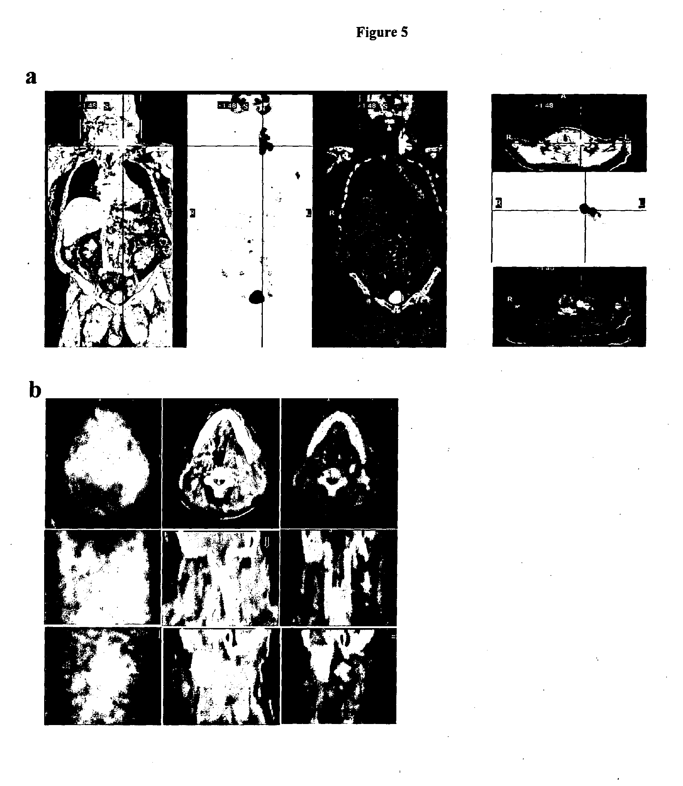

Fig. 5 A: ¹⁸F-FDG PET scans of a patient diagnosed with advanced small-cell lymphocytic leukemia.
   ¹⁸F-FDG PET scans demonstrate intense glucose metabolism in multiple enlarged lymph nodes, particularly in the left latero-cervical region. Coronal images are shown on the left and transaxial images of the cervical regions are displayed at the right side panel.
Fig. 5 B: Whole body scans and single photon emission computed tomography (SPECT) images of the same patient after iv injection of ¹³¹I-L19-SIP.
   The same patient received an intravenous infusion of 185 MBq and, subsequently, 5.55 GBq of ¹³¹I-LI9SIP. Transaxial, coronal, and sagittal SPECT-CT images of the cervical regions (1^{st}, 2^{nd} and 3^{rd} row, respectively) were acquired 8 days after the dose of 5.55 GBq. Left column shows scintigraphic images.

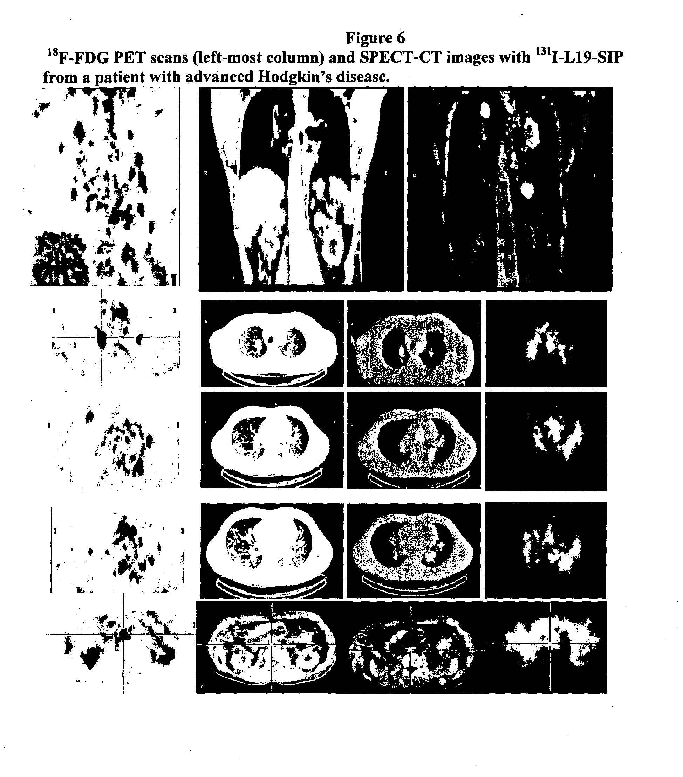

Fig. 6: ¹⁸F-FDG PET scans (left-most column) and whole body scans and single photon emission computed tomography (SPECT) images with ¹³¹I-L19-SIP from a patient with advanced Hodgkin lymphoma.
   ¹⁸F-FDG PET scans show intense glucose metabolism in multiple enlarged mediastinal lymph-nodes, intrapulmonary lesions (left-most column, first four images; intrapulmonary lesion marked), as well as in lumbo-aortic lymph-nodes (left-most image in lowest row). The same patient received intravenous injections of 185 MBq and, subsequently, 5.55 GBq of ¹³¹I-L19-SIP. SPECT-CT coronal (upper right panel) and transaxial images of the thorax (rows 2-4) as well as the upper-abdomen (lowest row), are shown, demonstrating selective uptake of ¹³¹I-L19SIP into the ¹⁸F-FDG avid lymphomatous lesions.

### Examples

### 1. Method of the invention

Tissue of F9 teratocarcinoma and of small cell kidney carcinoma was paraffin-embedded as described in the prior art. The tissue sections were placed into a steam-pressure cooker containing citrate buffer (0.01 M, pH 6.0), or EDTA solution (2 mM, pH 8.0), such that the tissue sections were fully submerged with the respective solution. The steam pressure cooker was heated up for about 5 minutes. Steam pressure cooking of paraffin-embedded tissues was performed at maximum heat (at about 125 °C and a pressure of about 100 kPa) for 2, 5, and 10 minutes for citrate and 1, 2, 5, and 10 minutes for the EDTA solution. For enzymatic antigen retrieval, paraffin sections were incubated for 15 minutes at 37 °C with protease type XIV (from streptomyces griseus, P5147-5G, SIGMA-Aldrich Chemie GmbH, Taufkirchen, Germany). After antigen retrieval, the paraffin-embedded tissue sections and non-pretreated were submitted to routine immunohistochemistry (IHC) staining according to the APAAP technology (Cordell et al, 1984), using ED-B antibody MX1 or the ED-B detecting fusion protein L19IL2 (Sauer et al, 2006).

It was surprisingly found that ED-B fibronectin was reliably and consistently detected on the paraffin-embedded specimen using a synthetic antibody or antibody fragment. Moreover, this was surprisingly achieved using a long steam cooking time of at least about 3 minutes and in the presence of EDTA. A skilled person would not have expected that IHC on paraffin-embedded tissue specimen is possible, and also he would not have applied the harsh parameters to biological probes.

In particular, after 2 minutes of steam-pressure cooking with EDTA (2 mM), ED-B antigen expression was not detectable on paraffin sections. Although 10 minutes of steam-pressure cooking was able to retrieve the ED-B antigen, this harsh procedure destroyed tissue integrity in such a way that ED-B expression could not be precisely attributed to histologically defined structures (e.g. blood vessels) anymore. Steam-pressure cooking with citrate resulted in insufficient antigen retrieval and thus, only a weak ED-B expression on paraffin sections could be found using IHC as compared with crystat sections. Also, antigen retrieval with proteinase XIV was an insufficient pretreatment of routine paraffin-embedded tissue sections to facilitate the detection of ED-B expression using IHC (APAAP technology).

### 2. Application of the method of the invention

### Materials and Methods

### Tissue specimens

Paraffin-embedded and shock-frozen tissue specimen of hematopoietic malignancies were drawn from the files of the Institute of Pathology, Campus Benjamin Franklin, Charité, Berlin, Germany. Altogether, more than 200 specimens of lymphomas of all common entities and leukemias were investigated. Additionally, 3 lymph node biopsies with the diagnosis of EBV-associated lymphoproliferation, 13 tonsils with follicular hyperplasia, 10 tumor-free lymph nodes harvested from the drainage of carcinomas, 12 enlarged lymph nodes with severe inflammation, and 9 bone marrow biopsies with non-neoplastic alterations were included in the study. Finally, frozen tissue specimens of 2 CLL, 6 mantle cell, and 3 follicular lymphomas were analyzed. The hematopoietic tumors were classified according to WHO classification Jaffe et al, 2001. Paraffin-embedded and frozen tissue specimens of 6 cases of clear cell renal cell carcinoma were included as positive controls.

The mouse embryonal teratocarcinoma cell line F9 was purchased from ATCC (Manas-sas, Virginia, USA). Athymic nude mice (8-week-old nude/nude CD1 mice, females) were obtained from Harlan, Italy (Correzzana, Milan, Italy). Nude mice were subcutaneously implanted with F9 cells as described (Carnemolla et al, 2002). F9 tumors, known for their strong ED-B FN expression, were harvested when approximately 10 mm in diameter and split in half. One half was paraffin-embedded, while the other half was shock-frozen in liquid nitrogen.

### Immunohistochemistry

To retrieve the ED-B FN epitope, paraffin-embedded tissue specimens were pretreated by steam pressure cooking in 2 mM EDTA, pH 8.0, 5 minutes. Immunohistochemistry of cryostat sections and pretreated paraffin sections was performed using the APAAP method (Cordell, 1984). ED-B FN was detected employing the dimeric L19IL2 fusion protein which consists of the human anti-ED-B scFv L19 fused to IL-2 (Pini et al, 1998; Viti et al, 1999 and MX1, an ED-B-specific murine IgG2a monoclonal antibody binding to a different epitope than L19. In each case, 10 microscopic fields were examined for ED-B FN expression in blood vessels and stroma at 400x magnification. ED-B FN expression was semi-quantitatively scored by assigning all cases to four categories based on the fraction of ED-B FN-positive blood vessels, i.e., more than 90%, between 50% - 90%, between 10% - 49%, or less than 10%, respectively. These scores were applied by 2 different investigators (S.S., H.D.), reaching best inter-observer agreement. For ED-B FN/CD34 double fluorescence staining, ED-B FN was detected by MX1 and goat anti-mouse Cy3-conjugated antisera (Dianova, Hamburg, Germany), employing a confocal microscope (Leitz, Wetzlar, Germany). After blocking the murine immunoglobulins with an excess of goat-anti-mouse serum, CD34 (QBEND, Immunotech, Marseille, France) was stained and subsequently detected by goat anti-mouse Cy2-conjugated antisera (Dianova). Nuclei were depicted by TOTO-3 (Molecular Probes, Paisley, UK).

Competition experiments were performed by titrating recombinant ED-B FN against MX1 mAb and L19IL2 constructs. Subsequently, the ED-B FN immunohistological staining properties of these mixtures were tested.

### RESULTS

### Method of the invention: ED-B FN detection in paraffin-embedded tissue specimens

MX1 and the fusion protein L19IL2 were used in the present method of the invention. Both anti-ED-B FN constructs detected the same ED-B FN expression pattern in paraffin-embedded and frozen tissue specimens of renal carcinoma (n=6), lymphocytic lymphoma (CLL; n=2), mantle cell lymphoma (n=6), and follicular lymphoma (n=3). Identical ED-B FN expression patterns were also found in the paraffin-embedded half and the frozen half of the split F9 tumor tissue specimens (Fig. 1).

To confirm the binding specificity of the MX1 mAb and the L19IL2 fusion protein, these reagents were pre-incubated with an excess of recombinant ED-B FN. The mixtures were incubated on human clear cell renal cell carcinomas and murine F9 teratocarcinoma with subsequent immunohistological detection of their binding pattern. Recombinant ED-B FN was capable of inhibiting the immunohistological binding of these reagents (Fig. 2).

Double stainings of diffuse large B cell lymphoma, marginal-zone lymphoma, lymphocytic lymphoma, plasmacytoma, follicular lymphoma (grade 1 and 3), and classical Hodgkin lymphoma (mixed cellularity and nodular sclerosis) revealed that ED-B FN is mostly co-localized with blood vessels, which were identified by their CD34-positive endothelia (Fig. 3). By investigating 1 normal lymph node, 1 lymph node with lymphadenopathy, 1 hyperplastic tonsil, and 2 normal bone marrow samples we also confirmed that the mostly small ED-B FN deposits are localized perivascular in non-malignant lymphoid and hematopoietic tissues.

This example proves the suitability of the method of the present invention for detecting a stromal epitope in paraffin-embedded sections.

### ED-B FN expression in normal and hyperplastic lymphoid tissue and normal bone marrow

Blood vessels of normal lymphoid tissue rarely and weakly express ED-B FN (Fig 4). ED-B FN expression in hyperplastic tonsils was heterogeneous. More than 90% of all blood vessels were ED-B FN-positive in 7/13 tonsils, whereas 5/13 tonsils show very low perivascular ED-B FN expression. Most of the ED-B FN-positive blood vessels of hyperplastic tonsils were found at the rim of germinal centers inside the inner mantle and marginal zone surrounding the highly proliferating follicular center cells. A few ED-B FN-positive vessels were situated in the fibrous fascicles of tonsils formed during chronic inflammation. ED-B FN expression was found in blood vessels of every diameter in lymphoid tissues.

Normal bone marrow samples revealed ED-B FN expression in less than 10% of all medium sized and large blood vessels. The few ED-B FN-positive medullar vessels bore a fibrous wall. ED-B FN expression was not found in normal sinusoids lacking fibrous support.

### ED-B FN expression in nodal and extranodal lymphomas

Nodal and extranodal lymphomas showed significantly more ED-B FN-positive blood vessels than normal lymphoid tissues (p<0.001). This finding was also true for both Non-Hodgkin and Hodgkin lymphomas. The degree of ED-B FN expression did not depend on the infiltrated organ, as lymphoma infiltration increased ED-B expression to almost the same extent in both nodal and parenchymal sites. High-grade malignant lymphomas (DLBCL, follicular lymphoma Grade 3, and ALL) exhibited significantly higher expression of ED-B FN in blood vessels than small B cell lymphomas (follicular lymphoma grade 1 and 2, CLL, mantle cell lymphoma, marginal cell lymphoma, plasmacytoma, and lymphoplasmocytic lymphoma; p=0.008). ED-B FN expression was found in blood vessels of all diameters and locations. Vessels inside the tumor, in the tumor stroma, and the tumor capsule bear ED-B FN, while ED-B FN expression was not detectable in vessels at the rim of tumor necrosis. ED-B FN expression did not significantly differ between classical Hodgkin lymphoma and lymphocytic-predominant Hodgkin lymphoma.

Three cases of EBV-associated lymphoproliferation (resembling diffuse large B cell lymphoma) showed high perivascular ED-B FN expression roughly to the same amount as in high-grade malignant lymphoma.

However, lymphoid tissue with severe inflammation, such as lymph nodes with lymphadenopathy or hyperplastic tonsils causing angina, revealed perivascular ED-B FN expression to almost the same extent as in malignant lymphoma.

### ED-B FN expression in neoplastic infiltrates of the bone marrow

In comparison to normal bone marrow, ED-B FN expression was enhanced in the bone marrow inflicted by myeloproliferative disease (p<0.001). The amount of ED-B FN-positive blood vessels did not significantly differ between acute myeloblastic leukemia and chronic myeloproliferative diseases, such as CML, polycythaemia vera, essential thrombocythaemia, or chronic idiopathic fibrosis.

Infiltration of bone marrow by plasmacytoma or lymphoma led to significantly increased ED-B FN expression in comparison to normal bone marrow (p<0.001).

ED-B FN expression in bone marrow biopsies infiltrated by hematologic malignancies was restricted to small and medium sized blood vessels consisting of an endothelial layer and a tiny wall of connective tissue. Sinusoids of the bone marrow were also ED-B FN-negative in the presence of neoplastic infiltrates.

### Immunofluorescence double stainings

To confirm perivascular ED-B FN expression, we performed immunofluorescence double stainings of ED-B FN and CD34-positive endothelia. These investigations indicate that ED-B FN is almost always associated with the wall of blood vessels and the perivascular connective tissue. In all tissue specimens of lymphoid and hematopoietic tissues, there was only a small amount of ED-B FN displaying an unequivocally extravascular localization. From the perspective of designing antibody-based tumor targeting therapies, ED-B FN in newly formed blood vessels is of particular relevance as compared to stromal ED-B FN, since it is easily accessible from the blood stream due to the frequent fenestrations and leakiness of tumor blood vessels (Cohen, 2007).

### ¹³¹I-L19SIP SPECT-CT images and ¹⁸F-FDG PET scans

As the radiopharmaceutical ¹³¹I-L19SIP contains the very same binding moiety, namely antibody fragment L19, used for immunohistochemical determination of ED-B FN in lymphoma samples, this radio labeled agent maintains both high-affinity for the target antigen and binding properties typical to other L 19 fusion proteins (Ebbinghaus, 2004). In the patient with advanced SLL NHL, transaxial, coronal and sagittal SPECT-CT images demonstrated selective ED-B FN targeting in a palpable, enlarged lymph node conglomerate in the left cervical region, which corresponded to high ¹⁸F-FDG uptake on the baseline PET-CT scan (Fig. 5). The absorbed dose in the target lesion was estimated at approximately 18 Gy, whereas it was 0.99 Gy and 0.42 Gy in the bone marrow and kidney, respectively. The patient was subsequently treated with a dose of 5.55 GBq of ¹³¹I-L19SIP.

In the patient with relapsed NS HL who similarly showed favorable lesion/bone-marrow dosimetry estimates, the absorbed dose of radioactivity in the target lesion - an intrapulmonary lymphoma lesion - was estimated to be approximately 14 Gy. The respective absorbed dose to the red bone marrow was calculated to be 1.3 Gy. In this patient the SPECT-CT images acquired 12 days after a dose of 5.55 GBq of ¹³¹I-L19SIP demonstrated selective ED-B FN targeting in multiple parenchymal lung lesions and in enlarged supraclavicular and lumbo-aortic lymph nodes with all such sites corresponding to high ¹⁸F-FDG uptake on baseline PET-CT scans (Fig. 6).

This experiment shows that ED-B fibronectin (FN) can also be detected in vivo using a labeled L19 antibody in SIP format, which is consistent with the results from the IHC method according to the invention. The patients were then treated with a therapeutic agent also targeting ED-B FN. In this case, both the diagnostic and therapeutic agent is identical.

### References:

Alessi P, Ebbinghaus C, Neri D. Molecular targeting of angiogenesis. Biochim Biophys Acta 2004;654:39-49.
Binz HK, Amstutz P, Plueckthun A. Engeneering novel nonimmunoglobuline domains. Nat Biotechnol 2005;23:1257-68.
Borsi L, Balza E, Bestagno M, Castellani P, Carnemolla B, Biro A, Leprini A, Sepul-veda J, Burrone O, Neri D, Zardi L. Selective targeting of tumour vasculature: comparison of different formats of an antibody (L19) to the ED-B domain of fibronectin. Int J Cancer 2002;102:75-85.
Carnemolla B, Borsi L, Balza E, et al. Enhancement of the antitumor properties of interleukin-2 by its targeted delivery to the tumor blood vessel extracellular matrix. Blood. 2002;99:1659-65.
Chothia C, Novotny J, Bruccoleri R, Karplus M. Domain association in immunoglobulin molecules. The packaging of variable domains. J Mol Biol 1985;185:651-63.
Chothia C, Lesk AM. Canonical structures for the hypervariable regions of immunoglobulins. J Mol Biol 1987;196:901-17.
Clackson T, Hoogenboom HR, Griffiths AD, Winter G. Making antibody fragments using phage display libraries. Nature 1991;352:624-28.
Cohen MM, Jr. Vascular update: Morphogenesis, tumors, malformations, and molecular dimensions. Am J Medical Genetics A. 2007;140A:2013-2038.
Cordell J, Falini B, Erber ON, et al. Immunoenzymatic labeling of monoclonal antibodies using immune complexes of alkaline phosphatase and monoclonal anti-alkaline phosphatase (APAAP complexes). J Histochem Cytochem. 1984;32:219-29.
De Candia LM, Rogers RJ. Characterization of the expression of the alternative splicing of the ED-A, ED-B, and V-regions of fibronectin mRNA in bovine ovarian follicles and corpora lutea. Repr Fert Dev. 1999;11:367-77
Dewhirst MW, Tso CY, Oliver R, et al. Morphologic and hemodynamic comparison of tumor and healing normal tissue microvasculature. Int J Radiat Oncol Biol Phys 1989;17:91-9.
Ebbinghaus C, Scheuermann J, Neri D, Elia G. Diagnostic and therapeutic applications of recombinant antibodies: targeting the extradomain B of fibronectin, a marker of tumor angiogenesis. Curr Pharm Des. 2004;10:1537-49.
Eberhard A, Kahlert S, Goede V, et al. Heterogeneiity of angiogenesis and blood vessel maturation in human tumors: implication for angiogenetic tumor therapies. Cancer Res 2000;60:1388-93.
Folkman J. The role of angiogensis in tumor growth. Semin Cancer Biol 1992;3:65-71 (Review).
Grabulovski D, Kaspar M, Neri D. A novel, non-immunogenic Fyn SH3-derived binding protein with tumor vascular targeting properties. J Biol Chem 2007;282:3196-204.
Holliger P, Prospero T, Winter G. "Diabodies": small bivalent and bispecific antibody fragments. Proc Natl Acad Sci USA 1993;90:6444-8.
Jaffe ES, Harris NL, Stein H, Vardiman JM (eds., 2001) World Health Organization Classification of Tumours, Pathology & Genetics: Tumours of Haematopoietic and Lymphoid Tissues. IARC Press, Lyon
Kabat et al., 1991, In: Sequences of Proteins of Immunological Interest, 5th Ed. Public Health Service, National Institutes of Health, Bethesda, MD.
Kaczmarek J et al, Distribution of Oncofetal Fibronectin Isoforms in Normal Hyperplastic and Neoplastic Human Breast Tissues, Int.J.Cancer:58, 11-16 (1994).
Kaspar M., Zardi L., Neri D., Fibronectin as target for tumor therapy. Int.J.Cancer: 118, 1331-1339
Köhler G, Milstein C. Continuous cultures of fused cells secreteing antibody of predefined specificity. Nature 1975;256:495-7.
Kriegsmann J, Berndt A, Hansen T, et al. Expression of fibronectin splice variants and oncofetal glycosylated fibronectin in the synovial membranes of patients with rheumatoid arthritis and osteoarthritis. Rheumatol Int. 2004;24:25-33.
Marks JD, Hoogenboom HR, Bonner TP, McCafferty J, Griffiths AD, Winter G. Bypassing immunization. Human antibodies from V-gene libraries displayed on phage. J Mol Biol 1991;222:581-97.
Matter CM, Schuler PK, Alessi P, et al. Molecular imaging of atherosclerotic plaques using a human antibody against the extra-domain B of fibronectin. Cir Res. 2004;95:1223-1233.
Morikawa S, Baluk P, Kaidoh T, et al. Abnormalities in pericytes on blood vessels and endothelial sprouts in tumors. Am J Pathol. 2002;160:985-00.
Pini A, Viti F, Santucci A, et al. Design and use of a phage display library. Human antibodies with subnanomolar affinity against a marker of angiogenesis eluted from a two-dimensional gel. J Biol Chem 1998;273:21769-76.
Pluckthun, 1994, In The Pharmacology of Monoclonal Antibodies, Vol. 113, Rosenburg and Moore eds., Springer-Verlag, New York, pp. 269-315
Rybak JN, Roesli C, Kaspar M, et al., The extra-domain A of Fibronectin is a vascular marker of solid tumors and metastases, Cancer Res 2007, 67: (22).10948 - 19957.
Santimaria M, Moscatelli G, Viale GL, et al. Immunoscintigraphic detection of the ED-B domain of fibronectin, a marker of angiogenesis, in patients with cancer. Clin Cancer Res. 2003;9:571-579.
Sauer S, Menrad A, Duerkop H, Menssen HD. Expression of ED-B fibronectin in human malignant lymphoma. Poster No LB-190, AACR meeting 2006.
Silacci M. et al, Potein Engineering, Design and Selection ,vol 19 no 10, pp 471-478, 2006.
Villa A, Trachsel E, Kaspar M et al., A high affinity human monoclonal antibody specific to the alternatively spliced EDA domain of fibronectin efficiently targets tumor ne-ovasulature in vivo, Int.J.Cancer 2008, 122, 2405-2413.
Viti F, Tarli L, Giovannoni L, et al. Increased binding affinity and valence of recombinant antibody fragments lead to improved targeting of tumoral angiogenesis. Cancer Res. 1999;59:347-52.
Zardi L, Carnemolla B, Siri A et al. Transformed human cells produce a new fibronectin isoform by alternative splicing of a previously unobserved exon. EMBO J. 1987;6:2337-2342.

## Claims

1. Method for detection of at least one stromal epitope and/or stromal protein, wherein
a) at least one tissue specimen is prepared as paraffin sections, and
b) at least one epitope is detected by immunohistochemistry,
**characterized in that**
step b) is performed by
i) steam pressure cooking over more than about 3 minutes, and
ii) in the presence of EDTA.

2. Method according to claim 1, wherein the result obtained in claim 1 is compared to at least one control tissue specimen.

3. Method of *in vitro* diagnosis of a disease, comprising performing the method according to claim 1 or 2, wherein the tissue specimen is obtained from a patient, and wherein the presence or increased presence or absence or reduced presence of the stromal protein comprising the epitope is indicative for the disease.

4. Method of determining whether a patient is responsive for a treatment, wherein a method according to claim 3 is performed, and wherein the stromal protein comprising the epitope is a stratification marker for the treatment.

5. Method of determining whether a patient is responsive for a treatment, wherein a method according to claim 3 is performed, and wherein the active compound used in the treatment binds to and/or acts on the stromal protein comprising the epitope.

6. Method according to any of claims 1 to 5, wherein the stromal epitope is part of an extracellular protein, in particular selected from ED-B fibronectin, ED-A fibronectin and Tenascin C.

7. Method according to claim 6, wherein the immunohistochemistry is performed using an antibody or antibody fragment or antibody mimetic specifically binding to the epitope.

8. Method according to claim 6, wherein the antibody is synthetic.

9. Method according to claim 1, wherein EDTA is present in a concentration of about 0,5 mM to 5 mM EDTA

10. Method according to claim 1, wherein steam pressure cooking is performed about 3 to 10 minutes, in particular about 4 to 7 minutes.

11. Method according to claim 1 or 10, wherein steam pressure cooking is performed for about 1 to about 10 minutes.

12. Method according to any of the previous claims wherein the temperature is about 110 °C to 135 °C and the pressure is about 50 to 150 kPA.
